# EUROPEAN PATENT SPECIFICATION

(11) **EP 2 094 300 B1**
(45) Date of publication and mention of the grant of the patent: **26.12.2012**
(21) Application number: 07736540.1
(22) Date of filing: 28.02.2007
(51) Int. Cl.: A61K 39/29, A61K 35/76, A61P 31/14

(54) **MEDICINAL FORMULATION FOR THE TREATMENT FOR HEPATITIS C**
MEDIZINISCHE FORMULIERUNG ZUR BEHANDLUNG VON HEPATITIS C
UTILISATION D'UNE FORMULATION MEDICAMENTEUSE DANS LE TRAITEMENT DE L'HEPATITE C

(30) Priority: 22.12.2006 IN MM21132006
(43) Date of publication of application: 02.09.2009
(73) Proprietor: Shah, Rajesh, Mumbai 400 089 MAH (IN)
(72) Inventor: Shah, Rajesh, Mumbai 400 089 MAH (IN)
(74) Representative: Williams, Paul Edwin
(86) International application number: PCT/IN2007/000079
(87) International publication number: WO 2008/078331

(56) References cited:
- EP-A1- 1 295 606
- WO-A1-97/08310
- RU-C1- 2 148 993
- DATABASE BIOSIS [Online] BIOSCIENCES INFORMATION SERVICE, PHILADELPHIA, PA, US; 1994, LIU FANG-HUA ET AL: "Preliminary studies on inactivation of serum HCV" XP002603725 Database accession no. PREV199497380172 & ZHONGHUA YUFANG YIXUE ZAZHI, vol. 28, no. 1, 1994, pages 3-5, ISSN: 0253-9624
- Peter Cornelius: "Nosoden und Begleitherapie" 1994, Pflaum-Verlag , München , XP002603726 ISBN: 3-7905-0706-7 * pages 29, 173 *
- "Homöopathisches Arzneibuch 1.Ausgabe 1978 Gesamtausgabe Amtliche Ausgabe" 1985, Deutscher Apotheker Verlag Stuttgart, Govi-Verlag GmbH, Frankfurt , Stuttgart, Frankfurt , XP002603727 ISBN: 3-7692-0932-X * page 80 - page 81 *
- Othon André Julian: "Neuere homöopathische Arzneimittelbilder" 1988, Johannes Sonntag Verlagsbuchhandlung GmbH Regensburg , Regensburg , XP002603728 ISBN: 3-87758-019-X * pages 3, 5 *
- LIU F. ET AL.: 'Preliminary Studies on Inactivation of Serum HCV' CHINESE PREVENTIVE MEDICINE JOURNAL vol. 28, no. 1, January 1994, pages 3 - 5, XP008130040

## Description

### Field of invention

This invention relates to medicinal formulations.

In particular, this invention relates to a medicinal formulation for the treatment for Hepatitis C.

### Definitions:

As used in the present specification, the following words and phrases are generally intended to have the meanings as set forth below, except to the extent that the context in which they are used indicates otherwise.

Antigenicity means the ability of a substance to trigger an immune response in a particular organism.

HCV is an acronym used for "Hepatitis C Virus".

Genotype means a specific genetic make-up (the specific genome) of a viral entity.

Potency means capacity of the formulation to produce strong immunological response and / or defense.

### Background:

### Introduction

Hepatitis is a liver disease. Amongst the known causative agents such as Viruses, drugs, toxins, and alcohol, viruses constitute the single most important cause of liver diseases. Though metabolic disorders are also known to be involved in precipitation of hepatitis, prevalence of such metabolic disorder related hepatic diseases is relatively low.

Viral hepatitis causes inflammation of the liver. The characteristic features of viral hepatitis are malaise, joint aches, abdominal pain, vomiting, defecation, loss of appetite, fever, enlarged liver and jaundice with peculiar symptoms such as dark urine, yellowing of eyes and skin.

Viral Hepatitis is further classified as Hepatitis A, B, C, E, and G depending on the type of viral agent involved. Furthermore mixed type of viral hepatitis for example Hepatitis B with C Hepatitis are also reported.

Each of the aforementioned viral agents has been characterized and depending on the type of viral agent involved the symptoms, mode of transmission, severity and nature (acute or chronic) of the infection vary. For example, hepatitis A (caused by piornovirus), which is usually acute, is contagious and can spread through personal contact, consumption of contaminated water or food and the infection while Hepatitis B (hepadnavirus) which can be acute or chronic, is transmitted through blood, tattoos, sexual intercourse, during child birth (from a mother to child).

Before the identification and characterization of the causative agent in 1989, Hepatitis C was referred to as parenterally transmitted "non A, non B hepatitis" (NANBH). Its discovery and characterization led to the understanding of its primary role in post-transfusion hepatitis and its tendency to induce persistent infection.

Hepatitis C virus (HCV) is an enveloped RNA virus in the Flaviviridae family which appears to have a narrow host range. Humans and chimpanzees are the only known species susceptible to infection, with both species developing similar disease.

The characteristic feature of this pathogen is the relative mutability of its genome, which is probably related to the high propensity (80%) of inducing chronic infection. HCV is clustered into several distinct genotypes which may be important in determining the severity of the disease and the response to treatment.

HCV is transmitted through the parenteral or percutaneous route, and replicates in hepatocytes.

The incubation period of HCV infection before the onset of clinical symptoms ranges from 15 to 150 days. In acute infections, the most common symptoms are fatigue and jaundice; but about 60 to 70% cases developing chronic infection, are asymptomatic.

Hepatitis C is a very lethal virus leading to liver cirrhosis and hepatocellular carcinoma leading to death. Cirrhosis develops in about 10% to 20% of persons with chronic infection, and liver cancer develops in 1% to 5% of persons with chronic infection over a period of 20 to 30 yearns. Liver cancer patients who do not have hepatitis B virus infection often show evidence of HCV infection. HCV infection intensifies the severity of underlying liver disease when it coexists with other hepatic conditions but how is yet not understood.

The spread of the HCV is primarily via blood transfusion, re-use of medical or surgical devices (such as in dialysis) and sexual contact. It tends to remain asymptomatic for 15 to 20 years in most cases. Hepatitis C candidates are more prone to contact hepatitis A and B leading to chronic hepatitis.

It is estimated that HCV affects 170 million people globally and 3 to 4 million persons are newly infected each year.

In India one fourth of all cases of chronic liver disease are caused by HCV. About 12.5 million people in India are HCV carriers and at least 25% of them are likely to develop chronic disease in near future. The prevalence Hepatitis C with HCV 3 genotype is particularly high in India and it requires at least a six month treatment regimen to eradicate the virus. But the chances of relapse are likely after stopping the treatment.

Not much progress has been achieved in the past 30 years in the treatment of liver diseases as compared with treatment of diseases in other therapeutic fields. Several drug candidates are now being evaluated, the progress is rather slow. New therapies are being vigorously sought.

Though various diagnostic methods like Enzyme immunosorbant assays (EIA), recombinant immunoblot assay (RIBA), polymerase chain reaction or PCR, branched DNA assay help in detecting HCV infection and its spread, the access to such methods is a major problem in developing countries. Furthermore, presently available treatment for chronic hepatitis C is too costly for most persons to afford.

Various drugs and methods for the treatment of viral hepatitis, in particular HCV, are available under the Modem as well as traditional system of medicines.

In mid-1990s, interferon-2a (INF-a) was the only available treatment with the success rate of only 10 to 20%. The poor response of this drug and therapy were attributed to HCV genotypes. There are six genotypes and more than 90 subtypes as of today. The genotypes are designated by numbers like 1 to 6, and the subtypes as a, b, c, and so on, in the order of their discovery. Quasispecies occur because HCV mutates freely, causing diverse genetic strains in each infected person. In America, Europe and Japan genotypes 1, 2 and 3 are most common. In case of HCV genotype 1 of United States and HCV genotype 3 of India showed similar kind of poor response. HCV 6 is restricted to South East Asia like China and India.

It is for this reason that the efficacy of modem drugs like interferon varies largely across different demographics and their use for treatment has not proved to be very successful.

Furthermore, interferons are always linked with deleterious side effects such as retinopathy, thyroiditis, acute pancreatitis, depression. Even newer versions of interferons like Pegylated interferon(PEG-Intron, Pegasys, Locteron) have not been able to alleviate the major drawbacks of this drug such as side effects, and poor response to HCV genotypes.

Because of the side effects associated with interferons, there are several limitations for use of interferons in many patients, for example Interferon-based therapy is problematic in patients with psychiatric disorders such as depression.

Still furthermore, high cost of this category of drugs make them inaccessible to large number people especially in the developing world.

Some of the antiviral agents, particularly nucleoside analogues such as Ribavirin, are active against HCV. However this category of drugs too suffer from the major drawback of various side effects such as anemia, fatigue, irritability, itching, skin rash, nasal stuffiness, sinusitis and cough which pose serious restrictions on the use of these drugs in majority of the patients.

Various combinations of antiviral agents with interferon and other agents such as Lactoferrin (a glycoprotein belonging to the iron-binding trans-family) have been in use. The success rate of the treatment is dependent on the genotype of the virus.

Several synthetic and biological drug candidates showing activity against Hepatitis C are disclosed in US patent applications US20070037867, US20060258868 and in a PCT application WO2004108068.

Developments in making a vaccine or adequate post-exposure prophylaxis measures have been hampered by inadequate information on the protective cellular and humoral immune responses against HCV infection and lack of an effective protective immune response. Furthermore, the mechanism by which HCV establishes viral persistence and causes a high rate of chronic liver disease has not been thoroughly elucidated.

WO2004024182 patent describes a method for isolating HCV peptides useful in vaccination, especially HCV T cell epitopes. A Hepatitis C virus (HCV) vaccine comprising at least two epitopes is disclosed in US20070031446 patent application. Major draw back is difficulty in predicting T-cell epitopes within the sequence of a polypeptide and even computer algorithms for T-cell epitope prediction have a high rate of both false-negatives and false-positives. Cost and complexity are few of the additional drawbacks.

PCT/US02/32512 discloses a nucleic acid particularly useful as a component of an adenovector or DNA plasmid vaccine providing a broad range of antigens for generating an HCV specific cell mediated immune (CMI) response against HCV.

Herbs and their bioactive compounds possess hepatoprotective properties. However Herbs useful in treating HCV are very few. Glycyrrhizin, from *Glycyrrhiza glabra and Glycyrrhiza uralesis* root has anti-viral properties and the action is via endogenous interferon induction and hepatoprotective effect. (Shiki, Y. et al., J. Gastroenterol. Hepatol., 1992, 7, 12-16. ).

Several commercial formulations consisting of combinations of one or more plants like *Silybum marianus, Taraxacum officinale, Verbena officinalis* are available. Herb *Silybum marianus* protects the liver from damage by viruses and toxins including alcohol and drugs and is widely used for treating Hepatitis C. *Taraxacum officinale* improve bile flow and reduce inflammation associated with hepatitis and cirrhosis whereas *Verbena officinalis* is for treating inflammation of the gallbladder, useful for jaundice having ability to relieve tension and stress and relieve mild depression.

Other herbal formulations for treating HCV are CH100, Sho-saiko-to (TJ-9), oxymatrine.

Several herbal preparations for treatment of hepatitis are disclosed in following patent applications IN726/CHE/2005(Indian Application), US patent Applications US20070020348, US20040142051.

Homeopathy is a nontoxic form of medicine that was developed approximately 200 years ago. This system uses highly diluted pathogens or other potentially *toxic* substances as remedies. These remedies provoke healing responses in a person's *immune system* or provoke other body responses to treat the root causes of illnesses.

Over 350 homeopathic remedies listed in various homeopathic provings, books, journals that have helped in curing various hepatitis viral disorders. Kali carb (Kali carbonium) is a polycrest drug having broad spectrum action that influences multiple organs of the system including liver and is a commonly used drug for treating Hepatitis C. *Chelidonium Majus* is a popular homeopathic medicine useful for various forms of hepatitis.

For over two decades, homeopathic practitioners have suggested that serially agitated dilutions of infectious agents (called "nosodes") are effective in the prevention of infectious disease.

A 'nosode' is a homeopathic remedy prepared from a pathological specimen. The starting material for preparation of a nosode can be blood, pus, any other body secretion or excretion, or even a diseased fragment of tissue, such as a growth. Rabies nosode, for example, starts with the saliva of a rabid dog and is then "potentized".

The preparation of nosodes derives from *homotoxicology,* a type of homeopathic therapy created by Hans-Heinrich Reckeweg in Germany in the first part of 18th century.

Homeopathic medicine, since its inception under Dr Samuel Hahneman, M.D. at the beginning of the 19th century, follows the principle of "infinitesimals." From this notion, the dosages of nosodes are in very minute and diluted forms. Thus, nosodes are not nearly as harmful as the untreated pathological product.

A 'nosode' is similar to an "oral vaccine" in the sense that its purpose is to "immunize" the body against a specific disease. The major difference between a nosode and a vaccine is of course the extremely small quantity of antigenic material in a nosode.

In the case of nosodes from bacteria and viruses, the preparation introduces the molecular imprints of possible antigens and other constituents of the pathological agent to the immune system. The working of the nosode is based on the fact that the immune system is induced to develop a defence mechanism which is effective against variety of antigens with this kind of molecular imprint, without being exposed to the virulence of the living agent.

Nosodes are also used as inter-current remedies in the treatment of chronic diseases. This is the most common use of nosodes in Homeopathic practice. An active ingredient obtained by combining a substance extracted from the mouth secretions of special animals of the family Varanidae or Eublepharidae, with a mixture of homeopathic substances for treating hepatitis has been disclosed in WO2005023275.

Different compounds, compositions and formulations coming from streams of medicines such as pharmaceuticals, biopharmaceuticals, biologicals, herbal medicines, ayurveda for treatment of hepatitis C have been reported earlier, but these remedies suffer from the common drawbacks such as side effects, high complexity, high costs, and accessibility. The quest for a preparation for treatment of Hepatitis C is still on.

"Nosoden und Begleittherapie", Peter Cornelius, 1994, Pflaum Verlag discloses commercial preperations of Hepatitis C nosodes which were prepared in Germany.

The "Präparateliste Staufen-Pharma, Göppingen dated 24.1.2001" lists Hepatitis nosodes (D6 for globuli and D200 for solutions).

Therefore, a need exists for an effective medicine for treatment of HCV infection that overcomes the limitations of existing therapies and at the same time is safe, effective and economical.

### Object of The invention

It is an object of this invention to provide a novel, specific immunity enhancing medicinal formulation, so as to control and reduce Hepatitis C virus activities. This invention is aimed at reducing the viral load and in turn arresting the disease process.

The formulation is also useful in accordance with another aspect for the treatment of cirrhosis of liver and liver cancer.

This preparation is also aimed to be a potential vaccine against Hepatitis C.

Comparing with the conventional antiviral substances against Hepatitis C, the formulation in accordance with this invention is not only significantly cost-affective but is also free from adverse effects.

Another object of this invention is to provide a novel medicinal formulation offering better patient compliance and thereby minimizing the risk of multidrug-resistant type of infections.

### SUMMARY OF INVENTION

Envisaged in accordance with this invention is a medicinal formulation for inducing specific immune response in patients with Hepatitis C.

In particular, this invention envisages a novel medicinal formulation for control of viral multiplication, in turn reducing the viral load in the body and hence controlling the pathogenesis and various symptoms associated with Hepatitis C disease.

In accordance with another aspect of the invention there is provided a medicinal formulation which can be adapted for use as a vaccine against Hepatitis C virus.

In accordance with the present there is provided a composition for the treatment of Hepatitis C infection and a process for making the same.

In accordance with an aspect of the invention, there is provided a composition according to claim 1 containing at least two serially diluted and potentized substances selected from :
1. Hepatitis C Genotype 1
2. Hepatitis C Genotype 2
3. Hepatitis C Genotype 3
4. Hepatitis C Genotype 4
5. Hepatitis C Genotype 5; and
6. Hepatitis C Genotype 6
said dilution being effected in a vehicle selected from a group consisting of normal saline, distilled water and ethyl alcohol(90 to 100 %).

In accordance with still another aspect of the invention, there is provided a Composition wherein the respective strains of Hepatitis C Genotype 1, Genotype 2, Genotype 3, Genotype 4, Genotype 5 and Genotype 6 are obtained from the patients infected by the respective strains and who were tested negative for other viral infections.

For the composition of the present invention, each of the serially diluted substances are 5c dilutions of the primary cultures of each of the strains in the vehicle, each 'c' representing a dilution of the previous stage in the vehicle where the proportion of the culture to vehicle is typically in the range of 1:99 to 50:50, wherein the final serial dilution is 30 to 200 c.

Typically, the serial dilution is in the range of 6c to 10 million c.

The final serial dilution is 30 to 200c.

In accordance with still another aspect of the invention, there is provided a process for making a composition comprising the following steps:
i) obtaining Hepatitis C Genotype 1 (Serum);
ii) obtaining Hepatitis C Genotype 2 (Serum);
iii) obtaining Hepatitis C Genotype 3 (Serum);
iv) obtaining Hepatitis C Genotype 4 (Serum);
v) obtaining Hepatitis C Genotype 5 (Serum);
vi) obtaining Hepatitis C Genotype 6 (Serum);
vii) preparing primary serum containing the virus of Genotype 1, Genotype 2, Genotype 3, Genotype 4, Genotype 5 and Genotype 6 by diluting solutions containing the serum containing the specific virus with about twice the quantity of a vehicle selected from a group consisting of normal saline, distilled water and ethyl alcohol(90 to 100 %);
viii) serially diluting primary serum (containing the virus) of strains Genotype 1, 2, 3, 4, 5 and 6 with the vehicle in a ratio of culture to vehicle in the range of 1: 99 to 50:50 to obtain a 5c stage dilution of each of the Strains Genotype 1, Genotype 2, Genotype 3, Genotype 4, Genotype 5 and Genotype 6 with potentization at each stage;
ix) Selecting at least two 5c dilutions from a group of 5c dilutions consisting of Genotype 1, Genotype 2, Genotype 3, Genotype 4, Genotype 5 and Genotype 6 to obtain a Hepatitis C Genotype virus complex (HCGV); and
x) serially diluting with potentization, the Hepatitis C Genotype virus complex (HCGV) with the vehicle in a ratio of complex to vehicle in the range of 1: 99 to 50:50 to obtain diluted Hepatitis C Genotype virus complex (HCGV) 30 to 200c.

In accordance with still another aspect of the invention, there is provided a process for making a composition wherein potentization is typically effected by holding a bottle containing the diluted culture/s in a closed fist and striking the fist on a hard surface repeatedly at a regular frequency or by exercising similar powerful strokes using a mechanical device which can strike the bottle on a hard surface.

Typically, strokes are given about 10 times.

Typically, the bottle containing the preparation for stroking for potentization is a securely stoppered glass bottle.

Typically, a mechanical device is used to exert a force of at least 6 dynes rhythmically at a frequency of 10 strokes in two minutes.

### Description:

Hepatitis C Genotype virus complex includes a wide range of Hepatitis C virus, inclusive of common and rare variants.

The Hepatitis C virus is known to cause serious diseases in humans such as Acute and Chronic Hepatitis C, Cirrhosis of liver and Liver cancer. The currently known strains of Hepatitis C virus are classified as:
1. Genotype 1
2. Genotype 2
3. Genotype 3
4. Genotype 4
5. Genotype 5 and
6. Genotype 6

Six patients were selected such that each of them was qualitatively tested positive for Hepatitis C, and each with one of the Genotypes as indicated above. Each of them was confirmed negative for other known viral infections such as Hepatitis B and HIV, in particular and from other Genotypes of HCV. One ml serum of each of the above patient was separated from blood samples and mixed in a vial containing 10 ml distilled water. (It is known that the virus is found in the serum.), and thoroughly mixed by stirring for five minutes.

About 2 ml of the above mixed serum was then mixed with 10 ml of distilled water and carefully preserved for further processing.

### About the potency:

Hepatitis C Genotype virus complex (HCMGV) was prepared in a range from HCGV 1c to HCGV 10 MILLION c, where 'c' denotes centesimal potency. Homeopathic medicines prepared from the diseased body tissues or organisms are prescribed to patients in various potencies, as per the well defined parameters of potency selection. Some of the parameters may be described in brief hereunder:
1. **Age**: Younger patients with higher susceptibility may be prescribed medium to high potency such as 30c to 1000c.
2. **Functional pathology:** Patients with functional disorders are prescribed medium to high potency.
3. **Structural pathology:** Patients with structural pathology are prescribed low potency to start with.
4. **Nosode:** When used as a nosode, 30c potency is the ideal It can be stepped up slowly.
5. **Response:** In case there is no response or the remedy stops acting, the potency can be stepped up from 30c to 50c to 100c to 500c.
6. **Active pathology:** In case of active pathology such as tubercular cavities or ulcers, higher potencies should be avoided.
7. **Susceptibility:** Higher the susceptibility, higher the potency.
8. Newer medicines in their initial evaluation period are better prescribed in 30c potency, if they are prepared from organism, venoms or any toxic source.

### References:

1. J.T. Kent's Philosophy
2. M.L.Dhawale's Principles and Practice of Homoeopathy

It may be noted that homeopathic preparation in a potency higher than 14c are found to have no physical traces or any molecules of the original source. In other words, all preparations after 15c are free from toxic properties of the original substances.

Thus, 6 Primary sera containing the virus for 6 strains of Hepatitis C virus as 1 _{PC}, 2_{PC}, 3_{PC}, 4_{PC}, 5_{PC} and 6_{PC} are prepared. About (2ml) of Primary serum (2ml) is mixed with a vehicle selected from a group of vehicles consisting of normal saline, distilled water and ethyl alcohol(90 to 100 %). Preferably, ethyl alcohol (90 to 100 %) is used as a vehicle.

The proportion of mixing of primary culture with the vehicle can range from 1:99 to 50:50. Preferably 2ml of the primary culture is mixed with 90 ml of the vehicle.

Although the dilution represented by the term 'c' means a dilution of 1:99, for the purposes of this specification the term 'c' is deemed to mean any dilution in the range of 1:99 to 50:50

### Serial Dilution And Potentization :

Potentization is a mathematico-mechanical process for rendering inert or poisonous antigen containing pathological residues, to a state of physical solubility, physiological assimilability so as to enhance their therapeutic activity and harmlessness, for use as a healing remedy.

The primary object of potentization is to reduce all substances designed for therapeutic use to "a state of approximately perfect solution or complete ionization, which is fully accomplished only by infinite dilution." (Arrhenius.)

Each resulting diluted culture is potentized typically by stroking by holding the bottle in a closed fist and striking the fist on a hard surface repeatedly at a regular frequency or by exercising similar powerful stroke using a mechanical device which can strike a bottle on a hard surface. Such strokes are given about 10 times. This preparation is labeled as Strain 1c potency. Thus 6 different preparations of 1c are obtained and labeled as 1-1c, 2-1c, 3-1c, 4-1c, 5-1c and 6-1.

In accordance with the preferred embodiment of this invention, the bottle containing the preparation for stroking for potentization is a securely stoppered glass bottle and a mechanical device is adopted to exert a force of at least 6 dynes rhythmically at a frequency of 10 strokes in two minutes.

This procedure of serial dilution followed by stroking is repeated 4 times with each 1c potency preparations to obtain respective 5c preparations for each strain. These are thus labeled as 1-5c, 2-5c, 3-5c, 4-5c, 5-5c and 6-5c.

### Preparation of Hepatitis C Genotype virus complex (HCGV):

At this point, about 2ml liquid + 10 ml of distilled water as prepared above, which is a mix consisting of all the above stated genotypes of Hepatitis C virus is now subjected to serial dilutions and potentization to obtain preparations like with higher potencies like, HCGV 2c, HCGV 3c, HCGV 4c..... HCGV 1000c, HCGV 50000 and above.
A vehicle is selected from a group of vehicles consisting of normal saline, distilled water and ethyl alcohol. Preferably ethyl alcohol (90 to 100%) is used as a vehicle.

### Examples:

Provided herein below are some of the case illustrations for Hepatitis C Genotype virus complex (HCV).

### Comparative Case I: Diagnosis: Hepatitis C acute infection

A 25 year old male who had undergone a blood transfusion ten years ago, reported with peculiar symptoms of jaundice like yellowing of skin, dark yellow colored urine, fatigue and occasional mild fever.
On testing he was found to be HCV positive.
The viral load of the patient at the time of first reporting was 15,880,753 IU/ml.
He was given Hepatitis Genotype virus complex (HCGV) 30c, essentially consisting of Hepatitis C virus Genotype 3 for a period of initial two months. Reasonable improvement was noticed as far as the severity of symptoms like fatigue, mild fever and yellowing of skin was concerned. The patient was then shifted to Hepatitis genotype virus complex 50c, essentially consisting of Hepatitis C virus Genotype 3 for a period of two months.
The patient reported significant improvement and was devoid of any major symptoms except some weakness and fatigue. For further prophylaxis and prevention from relapse the patient was put on two months treatment with Hepatitis genotype virus complex 100c, essentially consisting of Hepatitis C virus Genotype 3.
At the end of 6 moths the patient was free from the symptoms and his viral load was 1,963,643 IU/ml. Percentage reduction in viral load over a period of 6 months treatment was 87.65%.

### Case II:

A 32 year old female reported with complaints of abdominal cramps, fatigue and bouts of vomiting. During the meeting it was discovered that her husband a renal patient had been diagnosed for Hepatitis C fifteen years before.
On testing her HCV infection was confirmed.
At the time of first meeting her viral load was 2,590,000 IU/ml HCV RNA.
She was given Hepatitis Genotype Viral complex 30c, essentially consisting of Genotype 3 and Genotype 1 for a period of 1 month after which the treatment was shifted to Hepatitis Genotype Viral complex 50c, essentially consisting of Genotype 3 and Genotype 1.
There was improvement in health and partial relief from the symptoms. Subsequently the patient was given Hepatitis Genotype Viral complex 100c, essentially consisting of Genotype 3 and Genotype 1 for a period of 1 month after which complete relief from the symptoms was reported by the patient.
Viral load at the end of 3 months of treatment was 430,000 IU/ml HCV RNA. The percentage reduction in viral load in the period of 3 months was 83.4%

### Case III:

A 40 year old diabetic patient reported with complaints of stomach pain, bouts of vomiting , extreme weakness, inability to stand, fever and most importantly clay colored stools.
On testing the patient was found to be HCV positive.
The viral load of the patient was 127000 IU/ml.
Treatment was started with a Hepatitis Genotype Viral complex 30c, consisting of Genotype 1, Genotype 3 and Genotype 6. for a period of one month.
After the initial one month treatment the patient was given Hepatitis Genotype Viral complex 50c, consisting of Genotype 1, Genotype 3 and Genotype 6. Subsequently, the patient was given Hepatitis Genotype Viral complex 100c, consisting of Genotype 1, Genotype 3 and Genotype 6.
At the end of 3 months the viral load of the patient was as low as 21200 IU/ml. The viral load of the patient was rechecked at the end of 6 months and it was less than the detectable level.

### Case IV:

A 30 year boy with history of drug abuse was investigated to rule out viral infections such as HIV and HCV. On testing the patient was found to be HCV positive. The viral load of the patient was 15,08,159 IU/ml.
The patient was underweight and was suffering from minor infection of common cold. He was given Hepatitis Genotype Viral complex 30c, consisting of Genotype 4, Genotype 3 and Genotype 5 for a period of initial 3 months.
Later the patient was given Hepatitis Genotype Viral complex 50c, consisting of Genotype 4, Genotype 3 and Genotype 5 for a period 3 months.

There was reasonable improvement in health and he had gained some weight as well. The patient was then given Hepatitis Genotype Viral complex 100c, consisting of Genotype 4, Genotype 3 and Genotype 5 for a further period 4 months.
At the end of 10 months the viral load of the patient was 3,70,271 IU/ml and the patient was completely free from any of the reported symptoms. The percentage reduction in viral load was 75.4% in 10months.

### Case V:

A 50 year old female reported with history of blood transfusion during child birth above 15 years back.
On testing the patient was found to be HCV positive.
The viral load of the patient was 2,33,000 IU/ml.
The treatment was started with Hepatitis Genotype Viral complex 30c, consisting of Genotype 4, Genotype 3 and Genotype 5 for initial 3 months. This was followed by Hepatitis Genotype Viral complex 50c, consisting of Genotype 4, Genotype 3 and Genotype 5 for further 3 months.
Subsequently the patient was given Hepatitis Genotype Viral complex 50c, consisting of Genotype 4, Genotype 3 and Genotype 5 for one month and the viral load of the patient was taken which was 1,29,815 IU/ml.
The percentage reduction in viral load was 44.29% in 7 months.

### Case VI:

A 67 year old male reported with complaints of fatigue, loss of appetite, giddiness, and fever.
On testing he was found to be HCV positive.
The viral load of the patient was 1785714 IU/ml.

The patient was given Hepatitis Genotype Viral complex 30c, consisting of Genotype. 4_{;} Genotype 3 and Genotype 5 and Genotype 2 for a period of two months followed by Hepatitis Genotype Viral complex 50c, consisting of Genotype 4, Genotype 3 and Genotype 5 and Genotype 2 for further period of two months.
The patient reported overall relief from the symptoms and he continued the treatment for further period of 2 months with Hepatitis Genotype Viral complex 100c, consisting of Genotype 4, Genotype 3 and Genotype 5 and Genotype 2.
At the end of the 6 months the viral load of the patient was 11,69,010 IU/ml. During the follow-up visit of the patient after 6 months from completion of the treatment his viral load was found out to be as low as 2,23,656 IU/ml.
The overall viral reduction in percentage was 87.48% in 6 months.

### Case VII:

A 32 year male reported with symptoms of pain in stomach, extreme fatigue and giddiness with bursts of vomiting.
On testing the patient was found to be HCV positive.
The viral load of the patient was 104129IU/ml.
The patient started the treatment with Hepatitis Genotype Viral complex 30c, consisting of Genotype 4, Genotype 3 and Genotype 5 and Genotype 1 for initial two months and he continued the treatment for further two months with Hepatitis Genotype Viral complex 50c, consisting of Genotype 4, Genotype 3 and Genotype 5 and Genotype 1. Later on the patient was switched to Hepatitis Genotype Viral complex 100c, consisting of Genotype 4, Genotype 3 and Genotype 5 and Genotype 1 for additional 3 months.

At the end of 7 months of treatment the viral load of the patient was below the detectable levels. Needless to mention, he was completely cured from all the symptoms.

### Case VIII:

A 55 year male, a habitual alcoholic, reported with complaints of abdominal cramps , fatigue, loss of appetite and mild fever. He was consuming commercially available herbal hepatoprotective tablets Liv 52 in large quantities for relief.
On testing he was found to be HCV positive.
The viral load of the patient was 2,57,00,000 IU/ml.
He began his initial two month treatment with Hepatitis Genotype Viral complex 30c, consisting of all the genotypes of HCV, namely Genotype 1, Genotype 2 , Genotype 3 and Genotype 4, Genotype 5 and Genotype 6 . This was followed by Hepatitis Genotype Viral complex 50c for two months and subsequently he was given Hepatitis Genotype Viral complex 100c for a period of 2 month.
One month before his prescribed treatment (at the end of 5^{th} month of treatment) his viral load was 1,06,00,000 IU/ml.
The viral load reduction in percentage was 58.75% in 5 months.

### Case IX:

A 43 year female reported with complaints of loss of appetite, giddiness, extreme weakness with yellowing of skin.
The patient was diagnosed for HCV, 5 years before and she had undergone treatment with interferon alpha for similar symptoms.
She was again tested HCV positive.
Viral load of the patient was 7,64,200 IU/ml.
The patient was given Hepatitis Genotype Viral complex 30c containing all the genotypes for a period of two months followed by Hepatitis Genotype
Viral complex 50c for a period of two months.
At the end of 3 months of treatment, the viral load of the patient was 3,78,100 IU/ml.
The reduction in viral load in percentage was 50.52 % in just 3 months.

### Advantage and Application of the new preparation HCMGV 3c, 6c to 50000c (and above):

These preparations are prepared from the viral strains but they lack the viral toxicity. Preliminary clinical investigations have shown that the formulations prepared according to the above process help to retain the capacity to induce immune response in the body, which may helps prevention and treatment of Hepatitis C disease syndrome.

While considerable emphasis has been placed herein on the specific steps of the preferred process, it will be appreciated that many steps can be made and that many changes can be made in the preferred steps without departing from the principles of the invention. These and other changes in the preferred steps of the invention will be apparent to those skilled in the art from the disclosure herein.

## Claims

1. A composition for use in the treatment of Hepatitis C infection comprising, a homogenized mixture of at least two serially diluted and potentizcd substances selected from:
1. Hepatitis C Genotype 1
2. Hepatitis C Genotype 2
3. Hepatitis C Genotype 3
4. Hepatitis C Genotype 4
5. Hepatitis C Genotype 5
6. Hepatitis C Genotype 6
said dilution being effected in a vehicle selected from a group consisting of normal saline? distilled water and ethyl alcohol (90 to 100 %); wherein each of the serially diluted substances are 5c dilutions of said Hepatitis C strains in the vehicle, each 'c' representing a dilution of the previous stage in the vehicle in the range of 1:99 to 50:50, wherein the final serial dilution is 30 to 200c.

2. A composition for use as claimed in claim 1, wherein the respective strains of Hepatitis C Genotype 1, Genotype 2, Genotype 3, Genotype 4, Genotype 5 and Genotype 6 arc obtained from patients infected by the respective strains and who tested negative for other viral infections.

3. A process for making a composition as claimed in claim 1 or 2, comprising the following steps:
i) obtaining Hepatitis C Genotype 1 (serum containing the virus);
ii) obtaining Hepatitis C Genotype 2 (serum containing the virus);
iii) obtaining Hepatitis C Genotype 3 (serum containing the virus);
iv) obtaining Hepatitis C Genotype 4 (serum containing the virus);
v) obtaining Hepatitis C Genotype 5 (serum containing the virus);
vi) obtaining Hepatitis C Genotype 6 (serum containing the virus);
vii) preparing first cultures of Genotype 1, Genotype 2, Genotype 3, Genotype 4, Genotype 5 and Genotype 6 by diluting solutions containing the serum with twice the quantity of a vehicle selected from a group consisting of normal saline, distilled water and ethyl alcohol (90 to 100 %);
viii) serially diluting the first cultures of Genotype 1, 2, 3, 4, 5 and 6 with the vehicle in a ratio of culture to vehicle in the range of 1:99 to 50:50 to obtain a 5c stage dilution of each of the Strains Genotype 1, Genotype 2, Genotype 3, Genotype 4, Genotype 5 and Genotype 6 with potentization at each stage;
ix) selecting at least two 5c of the dilutions from the group of 5c dilutions consisting of Genotype 1, Genotype 2, Genotype 3, Genotype 4, Genotype 5 and Genotype 6 to obtain a Hepatitis C Genotype virus complex (HCGV); and
x) serially diluting with potentization, the Hepatitis C multiple Genotype virus complex (HCMGV) with the vehicle in a ratio of complex to vehicle in the range of 1:99 to 50:50 to obtain diluted Hepatitis C Genotype virus complex (HCGV) 30 to 200c.

4. A process for making a composition as claimed in claim 3, wherein potentization is effected by holding a bottle containing the diluted culture in a closed fist and striking the fist on a hard surface repeatedly at a regular frequency or by exercising similar powerful stroke using a mechanical device which can strike a bottle on a hard surface.

5. A process for making a composition as claimed in claim 3, wherein strokes are given about 10 times.

6. A process for making a composition as claimed in claims 4 or 5, wherein the bottle containing the preparation for stroking for potentization is a securely stoppered glass bottle.

7. A process for making a composition as claimed in claims 3 to 6 in which a mechanical device is used to exert a force of at least 6 dynes rhythmically at a frequency of 10 strokes in two minutes.

## Patentansprüche

1. Eine Rezeptur zur Anwendung für die Behandlung von Hepatitis C Infektionen, bestehend aus einer homogenisierten Mischung von mindestens zwei serienmäßig verdünnten und potenzierten Substanzen, die aus Folgenden gewählt werden:
1. Hepatitis C Genotyp 1
2. Hepatitis C Genotyp 2
3. Hepatitis C Genotyp 3
4. Hepatitis C Genotyp 4
5. Hepatitis C Genotyp 5
6. Hepatitis C Genotyp 6
die Lösung wirkt in einem Trägerstoff, der aus einer Gruppe gewählt wird, die aus normaler Saline, destilliertem Wasser und Äthylalkohol (90 bis 100%) besteht; wobei jedes der serienmäßig verdünnten Substanzen 5c Verdünnungen der Hepatitis C Bakterienstämme in dem Trägerstoff sind, jedes "c" repräsentiert eine Verdünnung der vorherigen Stufe im Trägerstoff in dem Bereich von 1:99 bis 50:50, wobei die endgültige serienmäßige Verdünnung 30 zu 200c beträgt.

2. Eine Rezeptur zur Anwendung nach Anspruch 1, wobei die jeweiligen Bakterienstämme von Hepatitis C Genotyp 1, Genotyp 2, Genotyp 3, Genotyp 4, Genotyp 5 und Genotyp 6 von Patienten erhalten werden, die durch die jeweiligen Bakterienstämme infiziert wurden und die negativ auf andere virale Infektionen getestet wurden.

3. Ein Verfahren zur Herstellung einer Rezeptur nach Anspruch 1 oder 2, die folgenden Schritte umfasst:
i) Beschaffung von Hepatitis C Genotyp 1 (Serum, welches das Virus enthält);
ii) Beschaffung von Hepatitis C Genotyp 2 (Serum, welches das Virus enthält);
iii) Beschaffung von Hepatitis C Genotyp 3 (Serum, welches das Virus enthält);
iv) Beschaffung von Hepatitis C Genotyp 4 (Serum, welches das Virus enthält);
v) Beschaffung von Hepatitis C Genotyp 5 (Serum, welches das Virus enthält);
vi) Beschaffung von Hepatitis C Genotyp 6 (Serum, welches das Virus enthält);
vii) Vorbereitung erster Kulturen von Genotyp 1, Genotyp 2, Genotyp 3, Genotyp 4, Genotyp 5 und Genotyp 6 durch die Verdünnung von Lösungen, die das Serum mit doppelter Menge eines Trägerstoffes, der aus einer Gruppe gewählt wird, die aus normaler Saline, destilliertem Wasser und Äthylalkohol (90 bis 100%) besteht, enthalten;
vii) serienmäßige Verdünnung erster Kulturen von Genotyp 1, 2, 3, 4, 5, und 6 mit dem Trägerstoff in einem Verhältnis der Kultur zum Trägerstoff im Bereich von 1:99 bis 50:50, um eine 5c Verdünnungsphase jedes Bakterienstammes Genotyp 1, Genotyp 2, Genotyp 3, Genotyp 4, Genotyp 5 und Genotyp 6 mit Potenzierung in jeder Phase zu erhalten;
ix) Auswahl von mindestens zwei 5c der Verdünnungen aus der Gruppe von 5c Verdünnungen, die aus Genotyp 1, Genotyp 2, Genotyp 3, Genotyp 4, Genotyp 5 und Genotyp 6 bestehen, um einen Hepatitis C Genotyp Virus Komplex (HCGV) zu erhalten; und
x) serienmäßige Verdünnung mit Potenzierungen, das Hepatitis C multiple Genotyp Virus Komplex (HCMGV) mit dem Trägerstoff in einem Verhältnis des Komplexes zum Trägerstoff im Bereich von 1:99 bis 50:50, um einen verdünnten Hepatitis C Genotyp Virus Komplex (HCGV) 30 bis 200c zu erhalten.

4. Ein Verfahren zur Herstellung einer Rezeptur nach Anspruch 3, wobei die Potenzierung durchgeführt wird, indem eine Flasche mit verdünnter Kultur in der geschlossenen Faust gehalten wird und die Faust im regelmäßigem Rhythmus auf eine harte Oberfläche gestoßen wird oder indem ähnlich starke Stöße mit Hilfe einer mechanischen Einrichtung durchgeführt werden, die eine Flasche auf eine harte Oberfläche stoßen kann.

5. Ein Verfahren zur Herstellung einer Rezeptur nach Anspruch 3, wobei die Stöße etwa 10 Mal getätigt werden.

6. Ein Verfahren zur Herstellung einer Rezeptur nach Anspruch 4 oder 5, wobei die Flasche, welche das Präparat zum Stoßen der Potenzierung enthält, eine mit einem Stöpsel sicher verschlossene Glasflasche ist.

7. Ein Verfahren zur Herstellung einer Rezeptur nach Anspruch 3 bis 6, in dem eine mechanische Einrichtung genutzt wird, um eine Kraft von mindestens 6 Dyn rhythmisch mit einer Häufigkeit von 10 Stößen in zwei Minuten anzuwenden.

## Revendications

1. Une composition à utiliser dans le traitement d'infection à l'hépatite C, comprenant un mélange homogénéisé d'au moins deux substances homéopathiques et diluées en série, sélectionnées parmi:
1. L'hépatite C de génotype 1
2. L'hépatite C de génotype 2
3. L'hépatite C de génotype 3
4. L'hépatite C de génotype 4
5. L'hépatite C de génotype 5
6. L'hépatite C de génotype 6
ladite dilution étant réalisée dans un véhicule choisi parmi un groupe constitué de sérum physiologique, d'eau distillée et d'alcool éthylique (90 à 100%) ; dans laquelle chacune des substances diluées en série sont des dilutions 5c desdites souches de l'hépatite C dans le véhicule, chaque « c » représentant une dilution de l'étape précédente dans le véhicule de l'ordre de 1:99 à 50:50, dans laquelle la dilution finale en série est de 30 à 200c.

2. Une composition à utiliser selon la revendication 1, dans laquelle les souches respectives de l'hépatite C de génotype 1, de génotype 2, de génotype 3, de génotype 4, de génotype 5 et de génotype 6 sont obtenues à partir de patients infectés par les souches respectives et dont le résultat était négatif pour d'autres infections virales.

3. Un procédé de préparation d'une composition selon les revendications 1 ou 2, comprenant les étapes suivantes :
i) l'obtention de l'hépatite C de génotype 1 (sérum contenant le virus);
ii) l'obtention de l'hépatite C de génotype 2 (sérum contenant le virus);
iii) l'obtention de l'hépatite C de génotype 3 (sérum contenant le virus);
iv) l'obtention de l'hépatite C de génotype 4 (sérum contenant le virus);
v) l'obtention de l'hépatite C de génotype 5 (sérum contenant le virus);
vi) l'obtention de l'hépatite C de génotype 6 (sérum contenant le virus);
vii) la préparation des premières cultures de génotype 1, de génotype 2, de génotype 3, de génotype 4, de génotype 5 et 6 par dilution de solutions contenant le sérum avec une quantité double d'un véhicule choisi parmi le groupe constitué de sérum physiologique, d'eau distillée et d'alcool éthylique (90 à 100%) ;
viii) la dilution en série des premières cultures de génotype 1, 2, 3, 4, 5 et 6 avec le véhicule dans un rapport de culture au véhicule de l'ordre de 1:99 à 50:50 pour obtenir une étape de dilution de 5c pour chacune des couches de génotype 1, de génotype-2, de génotype 3, de génotype 4, de génotype 5 et de génotype 6 avec dynamisation à chaque étape ;
ix) la sélection d'au moins deux dilutions de 5c du groupe des dilutions de 5c constitué de génotype 1, de génotype 2, de génotype 3, de génotype 4, de génotype 5 et de génotype 6 pour obtenir un génotype du virus de l'hépatite complexe (HCGV) ; et
x) une dilution en série par dynamisation, le génotype multiple du virus de l'hépatite C complexe (HCMGV) par le véhicule dans un rapport de complexe à véhicule de l'ordre de 1:99 à 50:50 pour obtenir le génotype du virus de l'hépatite C complexe de 30 à 200c.

4. Un procédé de préparation d'une composition selon la revendication 3, dans lequel la dynamisation s'effectue en tenant une bouteille contenant la culture diluée dans un poing fermé et en frappant du poing sur une surface dure à plusieurs reprises et à une fréquence régulière ou en exerçant un coup puissant semblable, à l'aide d'un dispositif mécanique capable de frapper une bouteille sur une surface dure.

5. Un procédé de préparation d'une composition selon la revendication 3, dans lequel les coups sont portés environ 10 fois.

6. Un procédé de préparation d'une composition selon les revendications 4 ou 5, dans lequel la bouteille contenant la composition à frapper pour dynamisation est une bouteille en verre fermement bouchée.

7. Un procédé de préparation d'une composition selon les revendications 3 à 6, dans lequel un dispositif mécanique est utilisé pour exercer une force rythmique d'au moins de 6 dynes à une fréquence de 10 coups en deux minutes.
